(19) Europäisches Patentamt
European Patent Office
Office européen des brevets

(11) Veröffentlichungsnummer: **0 052 775**
**B1**

(12) # EUROPÄISCHE PATENTSCHRIFT

(45) Veröffentlichungstag der Patentschrift: **02.05.85**

(21) Anmeldenummer: **81108794.9**

(22) Anmeldetag: **23.10.81**

(51) Int. Cl.⁴: **C 07 C 47/277**, C 07 C 47/228, C 07 C 45/50, C 07 C 45/65, C 07 C 45/62, C 07 C 43/303, C 07 C 43/166

(54) **Verfahren zur Herstellung von Aldehyden und Zwischenprodukte.**

(30) Priorität: **21.11.80 CH 8621/80**

(43) Veröffentlichungstag der Anmeldung: **02.06.82 Patentblatt 82/22**

(45) Bekanntmachung des Hinweises auf die Patenterteilung: **02.05.85 Patentblatt 85/18**

(84) Benannte Vertragsstaaten: **CH DE FR GB LI NL**

(56) Entgegenhaltungen:
**EP - A - 0 008 022**
**EP - A - 0 008 459**
**DE - B - 1 593 663**
**DE - B - 2 235 466**
**DE - B - 2 849 742**

(73) Patentinhaber: **L. GIVAUDAN & CIE Société Anonyme, CH-1214 Vernier-Genève (CH)**

(72) Erfinder: **Crameri, Yvo, Dr., Hohestrasse 103, CH-4104 Oberwil (CH)**
Erfinder: **Ochsner, Paul Albert, Dr., 30 Avenue des Tilleuls, CH-1203 Genf (CH)**
Erfinder: **Schudel, Peter, Dr., Neugutstrasse 16, CH-8624-Grüt-Wetzikon (CH)**

(74) Vertreter: **Urech, Peter, Dr. et al, Grenzacherstrasse 124 Postfach 3255, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von Aldehyden.

Das Verfahren ist dadurch gekennzeichnet, dass man eine Verbindung der Formel

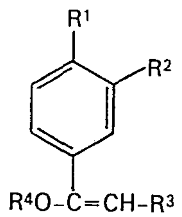

II

worin $R^1$ Isopropyl, n-Butyl, sec. Butyl, Isobutyl oder tert. Butyl oder Methoxy darstellt, $R^2$ Wasserstoff, oder $R^1$ zusammen mit $R^2$ Methylendioxy, $R^3$ Wasserstoff oder Methyl und $R^4$ Methyl oder Äthyl bedeuten, mit Kohlenmonoxyd und Wasserstoff bei erhöhter Temperatur und unter erhöhtem Druck in Gegenwart eines Rhodiumkatalysators zu einer Verbindung der Formel I, worin $R^1$, $R^2$, $R^3$ und $R^4$ obige Bedeutung besitzen, hydroformyliert, und, gegebenenfalls, eine erhaltene Verbindung der Formel I

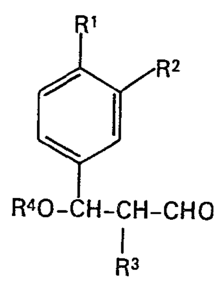

I

durch Abspaltung eines Alkohols $R^4OH$, z.B. säurekatalysierte Alkoholabspaltung, in eine Verbindung der Formel

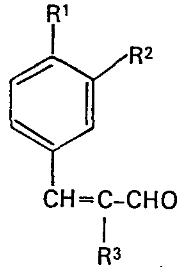

III

worin $R^1$, $R^2$ und $R^3$ obige Bedeutung besitzen, überführt und diese Verbindung der Formel III in an sich bekannter Weise zu einer Verbindung der Formel

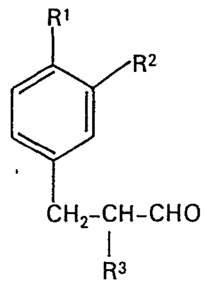

IV

worin $R^1$, $R^2$ und $R^3$ obige Bedeutung besitzen, hydriert.

Hydroformylierungen von Sauerstoffunktionen tragenden Ausgansmaterialien sind bekannt, siehe z.B.

J. Falbe, New Syntheses with Carbon Monoxide, Springer Verlag, Berlin/Heidelberg/New York, (1980), Seiten 122 und 123 und

Watanabe et al., Bulletin of the Chemical Society of Japan, 52 (9), (1979), Seiten 2735 und 2736.

Bei den bekannten Umsetzungen tritt jedoch die CHO-Gruppe ausnahmslos in α-Stellung zu der Sauerstoffunktion des Moleküls (bzw. an dem dem Sauerstoffatom nächstliegenden Kohlenstoffatom der Vinylgruppierung) ein, also nicht wie im erfindungsgemässen Fall in β-Stellung zu der Sauerstoffunktion.

Der Verbindungen I sind neue Zwischenprodukte.

Die Formel IV andererseits repräsentiert eine Klasse von bekannten Riechstoffen, wie z.B.

3-[p-Isopropylphenyl]-2-methyl-propionaldehyd (Cyclamen Aldehyd)
3-[p-tert.Butylphenyl]-2-methyl-propionaldehyd (Lilial)
3-[3,4-Methylendioxyphenyl]-2-methyl-propionaldehyd (Helional)
3-[p-Methoxyphenyl]-2-methyl-propionaldehyd (Canthoxal)
3-[p-tert. Butylphenyl]-propionaldehyd (Isolilal, Bourgeonal)
3-[p-n-Butylphenyl]-2-methyl-propionaldehyd
3-[p-iso-Butylphenyl]-2-methyl-propionaldehyd
3-[p-α-Methylpropyl-phenyl]-2-methyl-propionaldehyd.

Die erfindungsgemässe Hydroformylierung wird bewerkstelligt, indem Kohlenmonoxid und Wasserstoff, zweckmässigerweise im Volumenverhältnis von 1:4 bis 4:1, insbesondere im Volumenverhältnis von 1:3 bis 3:1 angewandt werden. Vorteilhaft verwendet man das genannte Gasgemisch im Überschuss, bezogen auf die eingesetzten Ausgangsstoffe der Formel II, z.B. bis zur 20–100fach molaren Menge.

Die Umsetzung wird vorteilhaft bei Temperaturen von 50 bis 150°C durchgeführt. Besonders bewährt haben sich Temperaturen von 90 bis 120°C.

Ferner führt man die Umsetzung zweckmässigerweise unter Drücken von 152–709,3 bar Überdruck (150 bis 700 atü) durch. Bevorzugt

wendet man Drücke von 253,3–506,2 bar Überdruck (250 bis 500 atü) an.

Die Umsetzung wird in Gegenwart von Rhodiumkatalysatoren durchgeführt. Als Katalysatoren kommen beispielsweise in Frage: Das reine Metall, Oxyde, Salze oder Komplexe, also z.B.

Rh/C, z.B. 5%
$RhCl_3 \cdot H_2O$
$Rh_2O_3$
$Rh(PPh_3)_3Cl$
$RhHCO\,(PPh_3)_3$
$RhCO(PPh_3)_2Cl$
$Rh_6(CO)_{16}$

Aus dieser Zusammenstellung ist ersichtlich, dass die Katalyse sowohl in homogener als auch in heterogener Phase durchgeführt werden kann. Bevorzugt ist die Durchführung in heterogener Phase. Vorteilhaft wendet man 0,01 bis 0,5 Gew.% Rhodium, berechnet als Metall, bezogen auf die eingesetzten Verbindungen der Formel II, an. Besonders bewährt haben sich Mengen von 0,03 bis 0,1 Gew.% Rhodium.

Das Verfahren nach der Erfindung führt man beispielsweise durch, indem man den Äther II, gegebenenfalls zusammen mit Lösungsmitteln, vorlegt, den Rhodium-Katalysator in den angegebenen Mengen einbringt, z.B. in einen Druckautoklaven, und unter Zuführung des Gemisches aus Kohlenmonoxid und Wasserstoff im angegebenen Verhältnis die Reaktion bei den beschriebenen Temperatur- und Druckbedingungen durchführt. In geeigneten Vorrichtungen lässt sich die Umsetzung aber auch vorteilhaft kontinuierlich durchführen. Nach dem Erkalten des Reaktionsprodukts wird entspannt und das überschüssige Gemisch aus Kohlenmonoxid und Wasserstoff abgetrennt und gegebenenfalls wieder verwendet, während der flüssige Reaktionsaustrag nach üblichen Methoden, z.B. durch Destillation, aufgearbeitet wird, wobei die Verbindung I als Hauptprodukt anfällt. Häufig ist es nicht erforderlich, diese Produkte in reiner Form zu isolieren. Für die weitere Verarbeitung genügt es, dann lediglich den Katalysator in einer ersten Destillation als Sumpf abzutrennen und das rohe Gemisch weiterzuverarbeiten.

Die β-Alkoxy-aldehyde I, die neu sind und ebenfalls Gegenstand der Erfindung bilden, können durch Alkoholabspaltung, z.B. säurekatalysierter Alkohol-Abspaltung in Verbindungen der Formel

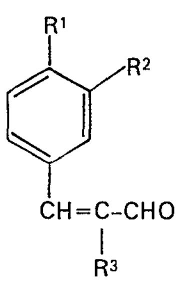

III

worin $R^1$, $R^2$ und $R^3$ obige Bedeutung besitzen, übergeführt werden.

Als Säuren eignen sich insbesondere Mineralsäuren, wie Salzsäure oder Schwefelsäure. Man arbeitet zweckmässigerweise bei Temperaturen zwischen 20 und 100°C, gegebenenfalls in Anwesenheit eines inerten Lösungsmittels, z.B. Toluol. Die Verbindung der Formel III wird hierauf in an sich bekannter Weise, siehe z.B. P. Bedoukian, Perfumery & Flavouring Synthetics, Elsevier, Amsterdam, London, New York, 1967, 151 oder US-PS 2 875 131 zu der Verbindung der Formel IV hydriert, also z.B. katalytisch unter Verwendung von Pd/Kohle.

Die Verbindungen der Formel II sind durch säurekatalysierte Elimination des Alkohols $R^4OH$ aus dem Ketal

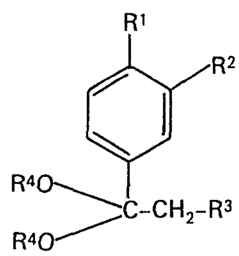

V

worin $R^1$, $R^2$, $R^3$ und $R^4$ obige Bedeutung besitzen, unter Abdestillieren des gebildeten Akohols $R^4OH$ zugänglich.

Die Destillation wird z.B. im Falle von Äthanol zweckmässigerweise bei Temperatur von 110 bis 170°C, bevorzugt bei 130–150°C, durchgeführt. Im Falle von Methanol sind auch etwas niedrigere Temperaturen möglich.

Die Verbindungen der Formel V sind durch Ketalisierung von Verbindungen der Formel

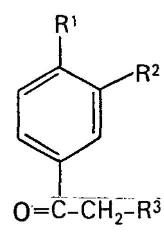

VI

worin $R^1$, $R^2$ und $R^3$ obige Bedeutung besitzen, herstellbar. Die Ketalisierung kann in an sich bekannter Weise durchgeführt werden, also z.B. durch Behandlung von VI mit Äthanol bzw. Methanol und ortho-Ameisensäureäthylester bzw. ortho-Ameisensäuremethylester in Anwesenheit von katalytischen Mengen von p-Toluol-sulfonsäure. Man arbeitet zweckmässigerweise bei Raumtemperatur oder leicht erhöhter Temperatur, z.B. bei 30–80°C.

Beispiel 1

a) In einen 2½-Liter-Sulfierkolben werden 1,050 Liter 1,2-Dichloräthan vorgelegt und hierauf 418 g Aluminiumchlorid eingetragen. Man kühlt auf 0°C ab und tropft innert 1¾ Stden 254 g Propionsäurechlorid bei 0–5°C zu (Eis/Aceton-Kühlung). Innert 30 Minuten lässt man auf 18° erwärmen und tropft nun bei 20° innert 2 Stunden 350 g tert.-Butylbenzol zu. Dabei wird mit Wasser leicht gekühlt. Man rührt 2 Stunden und lässt 12 Stunden stehen. Danach wird das Reaktionsgemisch unter Rühren auf ein Gemisch von 1,600 kg Eis, 260 ml Wasser und 260 ml Salzsäure conc. gegossen, wobei die Temperatur immer unterhalb 20° gehalten wird. Die wässrige Phase wird abgetrennt und 2 × mit 500 ml Dichloräthan extrahiert. Die vereinigten organischen Phasen werden 1 × mit 1 Liter Wasser, 3 × mit 400 ml Natronlauge 2% und 1 × mit 500 ml Wasser gewaschen (pH 6). Das Lösungsmittel wird am Rotavapor entfernt und der Rückstand (505 g) im Vakuum über eine 50 cm Kolonne destilliert.

Sdp. 0,0133 mbar (Sdp. 0,01 mmHg)
- 100° 50 g: Vorlauf
- 102° 425 g: p-tert.-Butyl-propiophenon, $n_D^{20}$: 1,5164

Rückstand 20 g
Siedepunkt der reinen Verbindung:
92–94°C/0,0533 mbar (0,04 mmHg);
$n_D^{20}$: 1,5184;
IR: Banden u.a. bei 1685 und 1605 $cm^{-1}$ und 800, 950 und 1225 $cm^{-1}$.

b) In einen 4½-Liter-Sulfierkolben werden 296 g abs. Alkohol und 6,5 g p-Toluolsulfonsäure vorgelegt. Es werden innert 30 Minuten 1223 g p-tert-Butyl-propiophenon zugetropft. Man gibt nun 953 g ortho-Ameisensäuretriäthylester zu. Die Temperatur wird mittels Wasserkühlung bei 30° gehalten. Es wird anschliessend noch 22 Stunden bei 20–25° nachgerührt. Das Reaktionsgemisch wird auf 31 ml Triäthylamin auf pH 8 gestellt und unter Rühren auf 300 ml Bicarbonatlösung 5% und 500 g Eis gegossen. Die wässrige Phase wird 1 × mit Äther extrahiert und die organische Phase mit 5%iger Bicarbonatlösung gewaschen. Nach Zugabe von 1 g Soda wird die Lösung am Rotavapor eingeengt.

Zur vollständigen Entfernung des Wassers werden anschliessend 500 ml Methylenchlorid zugegeben und es wird nochmals eingedampft.

Der Rückstand wird im Vakuum über eine 15 cm-Vigreuxkolonne unter Zusatz von 1 g Soda destilliert.

Sdp. 0,0667 mbar (0,05 mmHg)
- 92° 27 g: Vorlauf
~92° 1600 g: p-tert.-Butyl-propiophenon-diäthyl-ketal; $n_D^{20}$: 1,4821 (94,1%)

Rückstand 15 g
Siedepunkt der reinen Verbindung:
102°C/0,2667 mbar (0,2 mmHg);
$n_D^{20}$: 1,4830;
IR: Banden u.a. bei 835, 975, 1050, 1090, 1120 und 1170 $cm^{-1}$.

Bei der Destillation entsteht aus dem Äthylketal bereits etwas Enoläther.

c) In einer Destillationsapparatur, versehen mit einer 15 cm-Vigreuxkolonne, werden 500 g p-tert.-Butyl-propiophenon-diäthyl-ketal und 6 g para-Toluolsulfonsäure erhitzt (Ölbad 140°). Der gebildete Alkohol wird laufend abdestilliert. Innert 3 Stunden können 100 ml Alkohol abdestilliert werden. Nun wird am Wasserstrahlvakuum eingedampft und anschliessend am Hochvakuum destilliert.

Sdp. 0,0667 mbar (0,05 mmHg)
- 90° 10 g: Vorlauf
~91° 352 g: p-tert.-Butyl-propiophenon-enol-äthyläther, $n_D^{20}$: 1,5160 (85,2%)

Rückstand 55 g (polymerisiert).
Siedepunkt der reinen Verbindung:
93°/0,0133 mbar (0,01 mmHg);
$n_D^{20}$: 1,5153;
IR: Banden u.a. bei 850 und 1070 $cm^{-1}$.

d) In einem 4mal auf 1 mbar evakuierten und jeweils mit Argon begasten 500 ml-Uhde-Rührautoklaven wird eine Lösung von 0,135 g $Rh_6(CO)_{16}$ (0,127 mMol) und 109 g 83,3-proz. obiger Enoläther (416 mMol) in 100 ml Benzol unter Argon eingezogen. Mit Kohlenmonoxid und Wasserstoff im Verhältnis von 3:1 wird auf 240 bar komprimiert. Das Reaktionsgemisch wird unter Rühren auf 90°C erhitzt. Beim Aufheizen steigt der Druck bis auf 290 bar an.

Der Reaktionsverlauf wird gaschromatographisch verfolgt. Nach beendigter Reaktion wird das Reaktionsgemisch auf Raumtemperatur abgekühlt. Es wird aus dem Autoklaven gespült und im Rotationsverdampfer bei 60°C und am Wasserstrahlvakuum eingedampft. Das braune ölige Produkt (119,7 g) wird zwecks Abtrennung des Katalysators flachdestilliert (1 mbar, 125°C Badtemperatur) und ergibt 100,2 g Hydroformylierungsprodukt als gelbes Öl, das gemäss gaschromatographischer Analyse zu 62,1% aus der 2-Formylverbindung, nämlich dem 1-p-tert.-Butylphenyl-1-äthoxy-2-formyl-propan besteht. Daneben entsteht in geringer Menge das entsprechende 1-Formylderivat, nämlich das 1-p-tert.-Butylphenyl-1-äthoxy-1-formyl-propan.

Dieser Sachverhalt kann durch IR-Spektrum und NMR-Spektrum bestätigt werden.

e) 100,2 g destilliertes Hydroformylierungsgemisch werden mit 2 ml konz. Salzsäure (15,4 mMol) 6 Stunden bei 50°C gerührt.

Sobald die gesamte 2-Formylverbindung I umgesetzt ist (gaschromatographische Probe), wird der entstandene Äthylalkohol im Rotationsverdampfer bei 60°C und am Wasserstrahlvakuum abgedampft und das Reaktionsgemisch (91,4 g) unter Zugabe von 1,5 g Pyridin (90 mMol) neutralisiert und an einer 25 cm-Vigreux-Kolonne mit Vakuummantel im Hochvakuum (0,07 mbar) destilliert.

Man erhält 41,7 g 1-p-tert.-Butylphenyl-2-formyl-1-propen (Siedeintervall 75–80°C).

Smp. (aus Hexan) 68–70°C.

f) 1420 g 1-p-tert.Butylphenyl-2-formyl-1-pro-

pen gemäss Beispiel 1e) werden zusammen mit 6 g Pd/C (5%) und 6,8 g Soda in 20 ml Wasser bei 70–75°C im Rührautoklaven vermischt. Der Autoklav wird mit Wasserstoff beschickt [8,106 bar (8 Atm.)] und auf 110°C aufgeheizt. Nach 12 Stunden sind. ca. 230 Liter Wasserstoff absorbiert. Der rohe 3-(p-tert.-Butyl-phenyl)-2-methyl-propionaldehyd wird mit Äther versetzt, mit Wasser neutral gewaschen und über $Na_2SO_4$ getrocknet. Nach Entfernen des Äthers wird das Produkt im Vakuum fraktioniert destilliert. Man erhält dabei nach Abtrennen eines Vor- und eines Nachlaufs 1275 g 3-(p-tert.-Butyl-phenyl)-2-methyl-propionaldehyd in 95%iger Reinheit (ca. 88% d.Th).

Sdp. = 150–152°C/20 mbar (15 mmHg);
$n_D^{20}$: 1,5050.

Beispiel 2

a) p-Isopropyl-propiophenon

In einen Rundkolben, der mit Rührer, Thermometer, Kühler und Tropftrichter versehen ist, gibt man 525 ml 1,2-Dichloräthan und 209 g Aluminiumchlorid. Man kühlt auf 0°C ab und lässt bei 0–5°C innerhalb ¾ Stunden 127 g Propionsäurechlorid zutropfen. Nach der Zugabe lässt man die Temperatur bis auf +18°C steigen und addiert dann innerhalb 2 Stunden bei 20°C 157 g Cumol. Man lässt 12 Stunden stehen und giesst das Reaktionsprodukt dann auf ein Gemisch von 800 g Eis, 130 ml Wasser und 130 ml konz. Salzsäure. Die wässrige Phase wird abgetrennt und mit 2 × 250 ml Dichloräthan extrahiert. Die vereinigten organischen Phasen werden mit 3 × 200 ml 2%iger Natronlauge und 1 × 250 ml Wasser gewaschen. Das Lösungsmittel wird unter Vakuum abgedampft und der Rückstand (244 g) über eine 10 cm-Kolonne destilliert. Man erhält 192,1 g p-Isopropyl-propiophenon (Ausbeute: 83,2% der Theorie).

Sdp. = 76–77°C/0,2 mbar (0,15 mmHg);
$n_D^{20}$ = 1,5150;
$d_4^{20}$ = 0,9645.

b) p-Isopropyl-propiophenon-diäthyl-ketal

In einen Rundkolben, der mit Rührer, Thermometer, Kühler und Tropftrichter versehen ist, werden 48 ml absolutes Äthanol und 1 g p-Toluolsulfonsäure vorgelegt. Nun werden innerhalb 30 Minuten unter Rühren 181,5 g p-Isopropyl-propiophenon und anschliessend innerhalb 1 Stunde 152,5 g ortho-Ameisensäure-triäthylester zugetropft. Die Temperatur wird durch äussere Kühlung bei 30°C gehalten. Man rührt anschliessend noch 22 Stunden bei 20–25°C. Das Reaktionsgemisch wird alsdann mit 5 g Triäthylamin auf pH 8 eingestellt, hierauf auf 50 ml 5%ige Bicarbonatlösung und 80 g Eis gegossen. Die wässrige Phase wird abgetrennt und mit Äther extrahiert. Die vereinigten organischen Phasen werden mit 5%iger Bicarbonatlösung gewaschen; nach Zugabe von 0,3 g Natriumcarbonat wird das Lösungsmittel unter Vakuum abgedampft und der Rückstand über eine 15 cm-Kolonne destilliert. Man erhält 193,6 g p-Isopropyl-propiophenon-diäthylketal (Ausbeute: 75,1% der Theorie).

Sdp. = 66°C/0,0667 mbar (0,05 mmHg);
$n_D^{20}$ = 0,9305.

c) 1-p-Isopropylphenyl-1-äthoxy-1-propen

150 g p-Isopropyl-propiophenon-diäthylketal werden mit 1,5 g p-Toluolsulfonsäure versetzt und in einer Destillationsapparatur auf 140°C Ölbadtemperatur erhitzt. Es wird der gebildete Alkohol bei Atmosphärendruck laufend abdestilliert. Innert 3 Stunden werden so 20 g Äthanol abdestilliert. Nun wird am Wasserstrahlvakuum weiter eingedampft und anschliessend am Hochvakuum durch eine 30 cm-Kolonne destilliert. Man erhält 50,6 g 1-p-Isopropylphenyl-1-äthoxy-1-propen (Ausbeute: 41,4%).

Sdp. = 71–72°C/0,133 mbar (0,1 mmHg);
$n_D^{20}$ = 1,5093;
$d_4^{20}$ = 0,9324.

Die Hydroformylierung des Enoläthers wird bei 100°C, aber im übrigen wie im Beispiel 1 angegebenen durchgeführt. Dasselbe gilt für die Folgestufen, bei welchen ebenfalls die Bedingungen des Beispiels 1 angewandt werden können. Die Ausbeuten sind analog denjenigen des Beispiels 1.

Beispiel 3

a) p-Methoxy-propiophenon

In einen Rundkolben, der mit Rührer, Thermometer, Kühler und Tropftrichter versehen ist, gibt man 525 ml 1,2-Dichloräthan und 209 g Aluminiumchlorid. Man kühlt auf 0°C ab und lässt bei 0–5°C innerhalb 75 Minuten 127 g Propionsäurechlorid zutropfen. Nach der Zugabe lässt man die Temperatur auf 18°C steigen und gibt dann innerhalb 2 Stunden und bei 20°C 141,6 g Anisol zu. Man lässt 12 Stunden stehen und giesst alsdann das Reaktionsprodukt auf ein Gemisch von 800 g Eis, 130 ml Wasser und 130 ml konzentrierte Salzsäure. Die wässrige Phase wird abgetrennt und mit 2 × 250 ml 1,2-Dichloräthan extrahiert. Die vereinigten organischen Phasen werden mit 3 × 200 ml 2%iger Natronlauge und mit 250 ml Wasser gewaschen. Das Lösungsmittel wird unter Vakuum eingedampft und der Rückstand (198 g) über eine 25 cm-Kolonne destilliert. Man erhält 143 g p-Methoxy-propiophenon (Ausbeute: 66,4% der Theorie).

Sdp. = 90°C/0,533 mbar (0,4 mmHg);
Schmelzpunkt 26°C;
$n_D^{20}$ = 1,5451;
$d_4^{20}$ = 0,9645.

b) p-Methoxy-propiophenon-diäthyl-ketal

In einen Rundkolben, der mit Rührer, Thermometer, Kühler und Tropftrichter versehen ist, werden 40 ml absolutes Äthanol und 0,75 g p-Toluolsulfonsäure vorgelegt. Nun werden innerhalb 15 Minuten unter Rühren 120 g p-Methoxy-propiophenon und anschliessend innerhalb 1 Stunde 108 g ortho-Ameisensäuretriäthylester zugetropft. Die Temperatur wird unterhalb 30°C gehalten. Man rührt anschliessend noch 22 Stunden bei Raumtemperatur. Das Reaktionsgemisch wird mit 3,6 g Triäthylamin auf pH 8 eingestellt, dann auf 40 ml 5%iger Bicarbonatlösung und 60 g Eis gegossen. Die wässrige Phase wird abgetrennt und mit Äther extrahiert. Die vereinigten organischen Phasen werden mit 5%iger Bicarbonatlösung gewaschen; nach Zugabe von 0,2 g Natriumcarbonat wird das Lösungsmittel unter Vakuum abgedampft und der Rückstand über eine 15 cm-Kolonne destilliert. Man erhält 36,7 g-Methoxy-propiophenon-diäthyl-ketal (Ausbeute: 21% der Theorie).

Sdp. = 83–84°C/0,533 mbar (0,4 mmHg);
$n_D^{20} = 1,4858$;
$d_4^{20} = 0,9988$.

c) 1-p-Methoxyphenyl-1-äthoxy-1-propen

103,3 g p-Methoxy-propiophenon-diäthyl-ketal werden mit 1,1 g p-Toluolsulfonsäure versetzt und in einer Destillationsapparatur auf 140°C Ölbadtemperatur erhitzt. Dabei wird das gebildete Äthanol bei Atmosphärendruck laufend abdestilliert. Innert 3½ Stunden werden so 20 g Äthanol abdestilliert. Anschliessend wird am Hochvakuum über eine 15 cm-Kolonne destilliert. Man erhält 62,2 g 1-p-Methoxyphenyl-1-äthoxy-1-propen (Ausbeute: 74,8% der Theorie).

Sdp. = 78–79°C/0,4 mbar (0,3 mmHg);
$n_D^{20} = 1,5315$;
$d_4^{20} = 1,0175$.

Die Hydroformylierung wird bei 130°C, aber im übrigen wie im Beispiel 1 angegeben, durchgeführt. Dasselbe gilt für die Folgestufen. Die Ausbeuten sind analog.

**Patentansprüche**

1. Verfahren zur Herstellung von Aldehyden, dadurch gekennzeichnet, dass man eine Verbindung der Formel

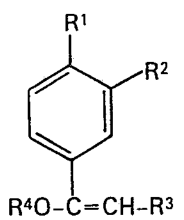

II

worin $R^1$ Isopropyl, n-Butyl, sec. Butyl, Isobutyl oder tert. Butyl oder Methoxy darstellt, $R^2$ Wasserstoff, oder $R^1$ zusammen mit $R^2$ Methylendioxy, $R^3$ Wasserstoff oder Methyl und $R^4$ Methyl oder Äthyl bedeuten, mit Kohlenmonoxyd und Wasserstoff bei erhöhter Temperatur und unter erhöhtem Druck in Gegenwart eines Rhodiumkatalysators hydroformyliert zu einer Verbindung der Formel

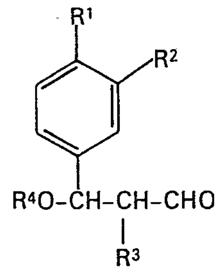

I

worin $R^1$, $R^2$, $R^3$ und $R^4$ obige Bedeutung besitzen, und, gegebenenfalls, eine erhaltene Verbindung der Formel I durch Abspaltung eines Alkohols $R^4OH$ in eine Verbindung der Formel

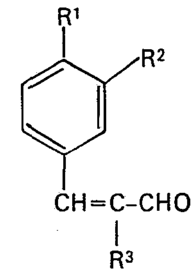

III

worin $R^1$, $R^2$ und $R^3$ obige Bedeutung besitzen, überführt und diese Verbindung der Formel III in an sich bekannter Weise zu einer Verbindung der Formel

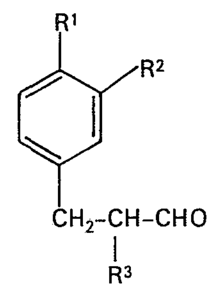

IV

worin $R^1$, $R^2$ und $R^3$ obige Bedeutung besitzen, hydriert.

2. Verfahren nach Anspruch 1, dadurch gekennzeichnet, dass man Temperaturen von 50° bis 150°C anwendet.

3. Verfahren nach Anspruch 1 oder 2, dadurch gekennzeichnet, dass man Drücke von 152–709,3 bar Überdruck (150 bis 700 atü) verwendet.

4. Verfahren nach einem der Ansprüche 1-3, dadurch gekennzeichnet, dass man die Katalyse in heterogener Phase durchführt.

5. Verfahren nach einem der Ansprüche 1-4, dadurch gekennzeichnet, dass man $Rh_6(CO)_{16}$ oder Rh/C als Katalysatoren verwendet.

6. Verbindungen der Formel

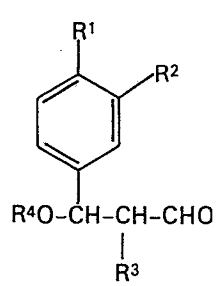

I

worin $R^1$ Isopropyl, n-Butyl, sec. Butyl, Isobutyl oder tert. Butyl oder Methoxy darstellt, $R^2$ Wasserstoff, oder $R^1$ zusammen mit $R^2$ Methylendioxy, $R^3$ Wasserstoff oder Methyl und $R^4$ Methyl oder Äthyl bedeuten.

7. 1-p-tert. Butylphenyl-1-äthoxy-2-formylpropan.

## Claims

1. A process for the manufacture of aldehydes, characterized by hydroformylating a compound of the formula

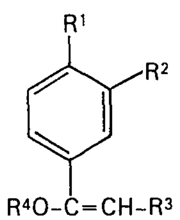

II

wherein $R^1$ represents isopropyl, n-butyl, sec. butyl, isobutyl or tert. butyl or methoxy, $R^2$ signifies hydrogen, or $R^1$ and $R^2$ together signify methylenedioxy, $R^3$ signifies hydrogen or methyl and $R^4$ signifies methyl or ethyl, with carbon monoxide and hydrogen at elevated temperature and under elevated pressure in the presence of a rhodium catalyst to give a compound of the formula

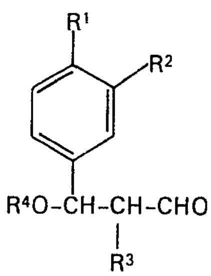

I

wherein $R^1$, $R^2$, $R^3$ and $R^4$ have the above significance, and, if desired, converting a compound of formula I obtained by cleavage of an alcohol $R^4OH$ into a compound of the formula

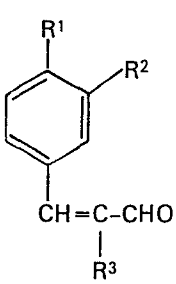

III

wherein $R^1$, $R^2$ and $R^3$ have the above significance, and hydrogenating this compound of formula III in a manner known per se to give a compound of the formula

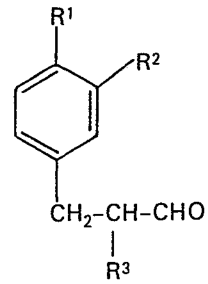

IV

wherein $R^1$, $R^2$ and $R^3$ have the above significance.

2. A process according to claim 1, characterized in that temperatures of 50° to 150°C are used.

3. A process according to claim 1 or 2, characterized in that pressures of 152–709,3 bar overpressure (150 to 700 atm) are used.

4. A process according to any one of claims 1–3, characterized in that the catalysis is carried out in the heterogeneous phase.

5. A process according to any one of claims 1–4, characterized in that $Rh_6(CO)_{16}$ or Rh/C is used as the catalyst.

6. Compounds of the formula

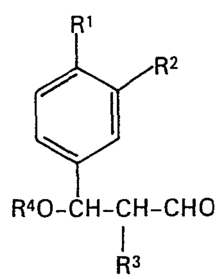

I

wherein $R^1$ represents isopropyl, n-butyl, sec. butyl, isobutyl or tert. butyl or methoxy, $R^2$ signifies hydrogen, or $R^1$ and $R^2$ together signify methylenedioxy, $R^3$ signifies hydrogen or methyl and $R^4$ signifies methyl or ethyl.

7. 1-p-Tert. butylphenyl-1-ethoxy-2-formylpropane.

## Revendications

1. Procédé de préparation d'aldéhydes, caractérisé en ce que l'on soumet un composé de formule

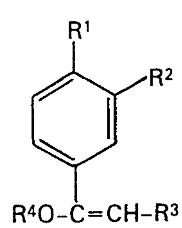

II

dans laquelle $R^1$ représente un groupe isopropyle, n-butyle, sec-butyle, isobutyle ou tert-butyle ou méthoxy, $R^2$ représente l'hydrogène, ou bien $R^1$ forme avec $R^2$ un groupe méthylènedioxy, $R^3$ représente l'hydrogène ou un groupe

méthyle et $R^4$ un groupe méthyle ou éthyle, à hydroformylation par l'oxyde de carbone et l'hydrogène à température élevée et pression élevée en présence d'un catalyseur au rhodium, ce qui donne un composé de formule

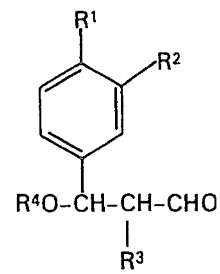

I

dans laquelle $R^1$, $R^2$, $R^3$ et $R^4$ ont les significations indiquées ci-dessus, et le cas échéant, on convertit un composé obtenu, répondant à la formule I, par séparation d'un alcool $R^4OH$, en un composé de formule

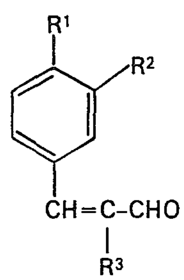

III

dans laquelle $R^1$, $R^2$ et $R^3$ ont les significations indiquées ci-dessus, et on convertit ce composé de formule III, par hydrogénation de manière connue en soi, en un composé de formule

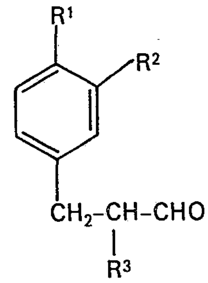

IV

dans laquelle $R^1$, $R^2$ et $R^3$ ont les significations indiquées ci-dessus.

2. Procédé selon la revendication 1, caractérisé en ce que l'on opère à des températures de 50 à 150°C.

3. Procédé selon la revendication 1 ou 2, caractérisé en ce que l'on opère à des surpressions de 152 à 709,3 bars (surpressions de 150 à 700 atmosphères °).

4. Procédé selon l'une des revendications 1 à 3, caractérisé en ce que l'on opère par catalyse en phase hétérogène.

5. Procédé selon l'une des revendications 1 à 4, caractérisé en ce que l'on utilise en tant que catalyseurs $Rh_6(CO)_{16}$ ou Rh/C.

6. Composés de formule

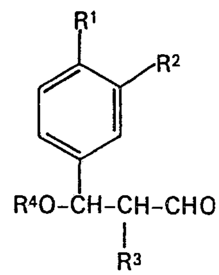

I

dans laquelle $R^1$ représente un groupe isopropyle, n-butyle, sec-butyle, isobutyle ou tert-butyle ou méthoxy, $R^2$ représente l'hydrogène ou bien $R^1$ forme avec $R^2$ un groupe méthylène-dioxy, $R^3$ représente l'hydrogène ou un groupe méthyle et $R^4$ représente un groupe méthyle ou éthyle.

7. Le 1-p-tert-butylphényl-1-éthoxy-2-formylpropane.